# EUROPÄISCHE PATENTSCHRIFT

(11) **EP 0 424 721 B1**
(45) Veröffentlichungstag und Bekanntmachung des Hinweises auf die Patenterteilung: **08.03.1995**
(21) Anmeldenummer: 90119299.7
(22) Anmeldetag: 09.10.1990
(51) Int. Cl.: H04N 5/235, A61B 1/04

(54) **Einrichtung zur automatischen Lichtversorgungsregelung von Endoskopen**
Automatic adjustment device for the light supply of an endoscope
Dispositif pour le réglage automatique de la source lumineuse d'un endoscope

(30) Priorität: 24.10.1989 DE 3935297
(43) Veröffentlichungstag der Anmeldung: 02.05.1991
(73) Patentinhaber: Richard Wolf GmbH, 75438 Knittlingen (DE)
(72) Erfinder: Ams, Felix, Ing., D-7539 Kämpfelbach (DE); Schäfer, Roland, D-7518 Bretten-Dürrenbüchig (DE)
(74) Vertreter: Wilcken, Thomas, Dipl.-Ing.

(56) Entgegenhaltungen:
- DE-A- 3 818 125
- GB-A- 2 149 264
- US-A- 4 025 955
- US-A- 4 399 466
- US-A- 4 638 366

## Beschreibung

Die Erfindung betrifft eine Einrichtung zur automatischen Regelung der für das Beleuchtungslicht eines Endoskopes vorgesehenen Lichtquelle mittels eines Videosignales, mit einem das Beleuchtungslicht übertragenden, an das zu betrachtende Objekt heranzuführenden Lichtleiter, einem mit einer Videokamera in Verbindung stehenden Bildleiter, einem Monitor zur Darstellung des von der Videokamera aufgenommenen Bildes und einer Signalverarbeitungsschaltung für das von der Videokamera erzeugte Signal.

Zur Betrachtung schwer zugänglicher Stellen, zum Beispiel Körperhöhlen, wird häufig ein Endoskop eingesetzt. Zur Auswertung und Dokumentation des Betrachteten besteht die Möglichkeit, durch eine außerhalb am Endoskop angebrachte Kamera Aufnahmen von den betrachteten Körperstellen zu machen. Bei Verwendung von Fotokameras können fotografische Aufnahmen gemacht werden und bei Einsatz eines Lichtwandlerelementes, beispielsweise eines CCD-Elementes, das die optischen Signale in elektrische Signale (Videosignale) umwandelt, können Aufzeichnungen mit einem Videorecorder hergestellt werden. Um bei solchen Aufnahmen gute Ergebnisse erzielen zu können, d.h. Bilder erzeugen zu können, die beispielsweise eine exakte Diagnose ermöglichen, muß dafür gesorgt werden, daß das interessierende Objekt optimal ausgeleuchtet wird. Wird von einer Lichtquelle eine konstante Lichtmenge geliefert, so kann es vorkommen, daß die betrachteten Körperstellen zum einen sehr stark reflektieren, also überstrahlt werden oder zum anderen zu schwach beleuchtet werden.

Es sind bereits Regelungen für Lichtquellen bekannt, bei denen ein von einer Bildaufnahmeeinrichtung erzeugtes Videosignal zur Regelung der Lichtmenge benutzt wird. So ist aus der DE-B- 31 18 341 eine Lichtregelung für ein Endoskop entnehmbar, bei dem die Bildaufnahme mit einer Fernsehkamera erfolgt. Hier wird die Lichtmenge, die auf den Lichtleiter trifft, so geregelt, daß unabhängig von Änderungen des Abstandes zwischen Beobachtungsobjekt und distalem Endoskopende die Spannung des Videosignals auf im wesentlichen konstantem Pegel (Spannungspegel) gehalten wird. Da bei dieser Form der Lichtregelung die Bildhelligkeit mit Hilfe des Mittelwertes des Videosignales geregelt wird, besteht der Nachteil dieser Lichtregelung darin, daß das interessierende Objekt nicht immer richtig beleuchtet wird.

Aus der DE-A- 37 43 090 ist weiter eine Beleuchtungsregelung für ein Endoskop bekannt, bei der das Beleuchtungslicht und die Helligkeit des abgebildeten Gegenstandes automatisch unabhängig von der durch den Bildleiter übertragbaren Bildgröße geregelt wird. Dabei wird der in Folge der Verwendung von Endoskopen mit unterschiedlichen Bildkreisdurchmessern und damit unterschiedlichen Bildbereichen veränderte Pegel des Videosignales dadurch berücksichtigt, daß die Bildbreite des endoskopischen Bildes Zeile für Zeile abgetastet und die größte Breite durch eine Spitzenwerthalteschaltung als Spannungspegel gespeichert und zum eigentlichen Ist-Wert des Videosignals addiert wird. Nachteilig ist hierbei, daß die größte Breite und nicht die Fläche des Bildes in die Regelung des Videosignals eingeht.

Weiter sei auf die DE-B- 35 09 825 hingewiesen, die eine Beleuchtungsregelung für einen Lichtprojektor offenbart. Diese Regelung erlaubt es, ein Objekt in seinem Abstand vom distalen Endoskopende richtig auszuleuchten und die Lichtmenge auf einen Maximalwert zu begrenzen. Durch einen Soll-Ist-Wert-Vergleich wird sichergestellt, daß die maximal zulässige Amplitude des Videosignals eingehalten wird. Diese Regelung verhindert zwar eine Überstrahlung der hellsten Bildteile, hat aber den Nachteil, daß der Bildhintergrund zu dunkel werden kann.

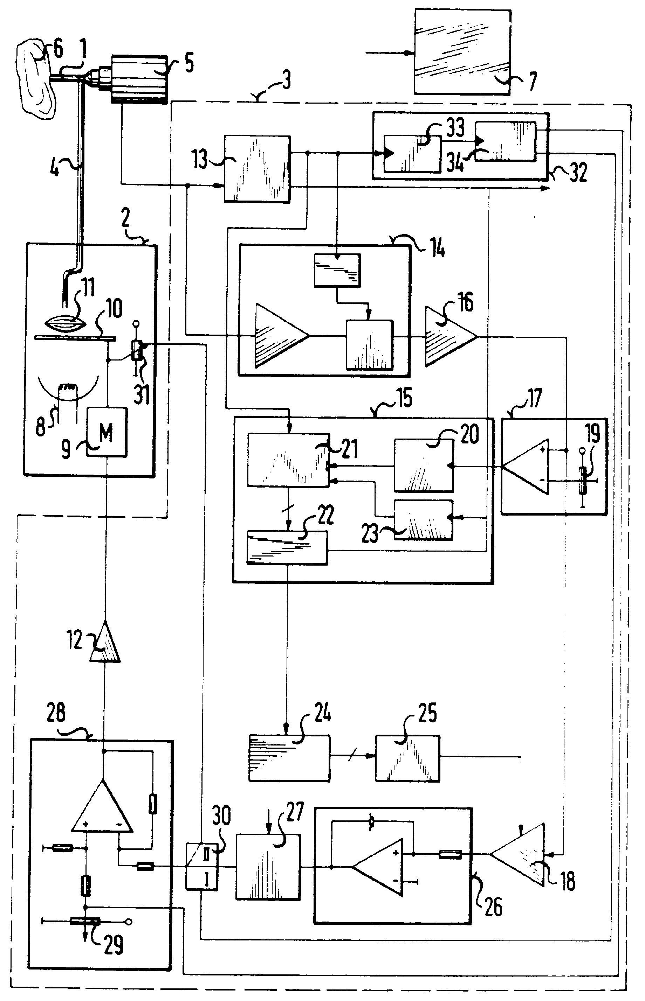

Eine weitere Einrichtung zur Lichtregelung für ein Endoskop ist schließlich noch der DE-A- 38 18 125 zu entnehmen. Bei dieser Einrichtung wird die Änderung des Bildkreisdurchmessers bei Verwendung von Endoskopen mit unterschiedlichen Durchmessern berücksichtigt und die Lichtmenge entsprechend geregelt. Dazu werden die Signalwerte von Bildelementen, sogenannten Pixeln, einer Festkörper-Bildaufnahmeeinrichtung darauf überprüft, ob sie einen festgelegten Dunkelwert überschreiten. Aus dem Ergebnis dieser Prüfung wird die der Querschnittsfläche des Bildleiters entsprechende Bildabtastfläche festgestellt und durch Auswählen eines Korrektursignals ein Lichtregelsignal erzeugt, durch das die dem Lichtleiter zugeführte Lichtmenge geregelt wird. Diese Lichtregeleinrichtung hat den Nachteil, daß die in der Zeichnung der OS gestrichelt gezeichnete Quadratfläche und nicht die eigentliche Bildkreisfläche des Endoskops zur Regelung des Videosignals benutzt wird.

Es ist daher die Aufgabe der Erfindung, eine automatische Regelung für das Beleuchtungslicht eines Endoskopes zu schaffen, die eine für Videoaufnahmen optimale Ausleuchtung des Beobachtungsobjektes sowohl bei variablen Objektabständen als auch unabhängig von dem jeweiligen Bildkreisdurchmesser des Endoskops sicherstellt.

Diese Aufgabe wird erfindungsgemäß dadurch gelöst, daß für die Erzeugung eines Ist-Wertes aus dem Videosignal die Signalverarbeitungseinrichtung einen Verstärker mit variabler Verstärkung, eine Integrationsschaltung und ein Abtast-Halteglied umfaßt sowie zur Erzeugung des zur Steuerung des Verstärkers benötigten Signals eine Einrichtung zur Erfassung der Zeilenanzahl des auf dem Monitor dargestellten Bildes und einen Funktionsbaustein zur Durchführung einer mathematischen Operation aufweist. Dabei kann die Einrichtung mit einem Wahlschalter für automatische oder manuelle Lichtregelung ausgestattet und eine Überwachungsschaltung vorgesehen sein, welche bei fehlendem Videosignal die Lichtquellenregelung auf manuellen Betrieb umschaltet und die Blende schließt. Auch können Mittel vorgesehen sein, mit deren Hilfe aus der Stellung der Blende die von der Lichtquelleneinrichtung abgegebene Lichtmenge ermittelt und angezeigt werden kann.

Die durch die Erfindung erreichten Vorteile sind im wesentlichen darin zu sehen, daß die Fläche des Bildes in die Regelung eingeht, was ebenso zu einer besseren Ausleuchtung führt, wie die Möglichkeit, die mathematische Operation mit einer geeigneten mathematischen Funktion ausführen zu können.

Die Erfindung ist nachstehend anhand eines in der Zeichnung dargestellten Ausführungsbeispieles näher erläutert.

Die Gesamtanordnung der erfindungsgemäßen Einrichtung umfaßt im wesentlichen ein Endoskop 1, eine dasselbe mit Beleuchtungslicht versorgende Lichtquelle 2 und eine Signalverarbeitungseinrichtung 3. Zur Leitung des Beleuchtungslichtes an das distale Ende des Endoskopes 1 ist ein dieses mit der Lichtquelle 2 verbindender Lichtleiter 4 vorgesehen. Das Endoskop 1 ist mit einer Videokamera 5 ausgerüstet, die das von dem Endoskop 1 weitergeleitete Bild, beispielsweise aus einer Körperhöhle 6, aufnimmt, in ein Videosignal umwandelt und der Signalverarbeitungseinrichtung 3 sowie einer Anzeigevorrichtung 7 zuführt. Auf dieser wird das Videosignal in Form eines Bildes des betrachteten Gegenstandes dargestellt.

Die Lichtquelle 2 umfaßt eine Lampe 8, einen Motor 9, eine Blende 10 und eine Einrichtung 11 zur Fokussierung und Filterung des von der Lampe 8 abgegebenen Lichtes, das dann dem Lichtleiter 4 zugeführt wird. Der Motor 9 der Lichtquelle 2 dient der Betätigung der Blende 10, die je nach benötigter Lichtmenge geöffnet oder geschlossen wird. Die hierzu benötigten Steuersignale werden in der Signalverarbeitungseinrichtung 3 erzeugt und über einen Motortreiber 12 zur Steuerung von Drehgeschwindigkeit und Drehrichtung des Motors an diesen ausgegeben.

In der Signalverarbeitungseinrichtung 3 werden durch eine Synchronabtrennstufe 13 das Horizontal- und Vertikal-Synchronsignal aus dem ankommenden Videosignal herausgefiltert und einer Zählschaltung 15, einer Überwachungsschaltung 32 sowie einer Schaltung 14 zur Definition des Schwarzwertes des Videosignals jeder Zeile des Kamerabildes zugeführt. Das von der Schaltung 14 abgegebene Videosignal wird über einen Verstärker 16 einem Komparator 17 zugeleitet und gelangt gleichzeitig zu einem Verstärker 18 mit variabler Verstärkung.

Der Komparator 17 vergleicht das Videosignal mit einer mittels eines Reglers 19 einstellbaren Helligkeitsschwellspannung. Ist die Bildhelligkeit in einer Zeile des Kamerabildes größer als der durch die Helligkeitsschwellspannung repräsentierte Helligkeitswert, so wird in der Zählschaltung 15 ein Monoflop 20 getriggert, das für eine Zeilenperiode den Zähler 21 der Zählschaltung 15 freigibt, so daß durch das Horizontal-Synchronsignal der Zählerstand um 1 erhöht werden kann. Auf diese Weise werden die Bildzeilen gezählt, die eine Bildinformation enthalten, deren Helligkeitswert größer ist als der eingestellte Helligkeitsschwellwert. Zeilen mit unter dem Helligkeitsschwellwert liegendem Helligkeitswert bleiben unberücksichtigt und gehen daher nicht mit in die Helligkeitsregelung ein. Mit Hilfe des Vertikal-Synchronsignals am Ende der letzten Bildzeile wird der Zählerstand in eine Schaltung 22 zur vorübergehenden Datenspeicherung (Latch) übernommen und der Zähler 21 durch ein zeitverzögernd schaltendes, vom Vertikal-Synchronsignal getriggertes Monoflop 23 zurückgesetzt.

Das Zählergebnis wird einem programmierbaren Festwertspeicher 24, zum Beispiel einem EPROM, zugeführt. Der Zuordnung zwischen den in diesem Speicherbaustein 24 abgelegten Daten und der Zeilenanzahl (Adressen) liegt hierbei eine Gesetzmäßigkeit zugrunde, die sich mathematisch durch die Funktion f(x)=1/x² beschreiben läßt. Der Ausgang des programmierbaren Festwertspeichers 24 ist auf einen Digital/Analog-Wandler 25 geführt, der ein analoges Signal bildet, das dem Verstärker 18 zugeführt wird und dessen Verstärkung so steuert, daß diese umso kleiner wird, je größer die Fläche der hellen Teile des Kamerabildes ist.

Das Ausgangssignal des Verstärkers 18 wird in einer Integrationsschaltung 26 über eine Bildperiode integriert und das Integrationsergebnis durch ein Abtast-Halteglied 27 gespeichert, das mittels des Vertikal-Synchronsignals am Ende der letzten Bildzeile getriggert wird. Somit wird der aus dem Videosignal durch den Verstärker 18, den Integrator 26 und das Abtast-Halte-Glied 27 gebildete Ist-Wert unabhängig von der Fläche der hellen Bildteile und damit von dem Bildkreisdurchmesser des Endoskopes. Am Ausgang des Abtast-Haltegliedes 27 steht das Integrations-ergebnis als Ist-Wert für einen Soll-Ist-Wert-Vergleicher 28 zur Verfügung, der entsprechend dem Ergebnis des Vergleiches von Ist-Wert und mittels eines Reglers 29 eingestelltem Soll-Wert ein Steuersignal an den Motortreiber 12 gibt, der den Motor 9 der Lichtquelle 2 steuert.

Bei der erfindungsgemäßen automatischen Lichtregelung für Endoskope kann anstatt des automatischen Betriebes, d.h. der Lichtregelung über das Videosignal, auch manueller Betrieb, also Einstellen der Beleuchtungsstärke durch Betätigen eines Reglers von Hand, gewählt werden, wodurch die abgegebene Lichtmenge individuell vom Anwender eingestellt werden kann. Hierzu enthält die Signalverarbeitungseinrichtung 3 einen Umschalter 30, der vom Anwender von automatischer Lichtmengenregelung (Stellung I) auf manuelle Lichtmengenregelung (Stellung II) umgeschaltet werden kann. Ist die Betriebsart manuelle Lichtmengenregelung gewählt, so wird der Ist-Wert für den Soll-Ist-Wert-Vergleich über den Ist-Wert-Geber 31, der bspw. als mit der Welle der Blende 10 mechanisch gekoppeltes Potentiometer ausgeführt sein kann, dem Soll-Ist-Wert-Vergleicher 28 zugeführt. Der Soll-Wert wird hierbei weiterhin über den Regler 29 eingestellt und das Ausgangssignal des Soll-Ist-Wert-Vergleichers 28 steuert über den Motortreiber 12 den Motor 9 der Lichtquelle 2.

Weiter ist die automatische Lichtquellenregelung mit einer Überwachungsschaltung 32 ausgestattet, die bei fehlendem Videosignal dafür sorgt, daß der Umschalter 30 von automatischem auf manuellen Betrieb umschaltet und die Blende 10 der Lichtquelle geschlossen wird. Damit wird vermieden, daß bspw. bei nicht angeschlossener Kamera der Anwender geblendet wird oder daß eine hohe Wärmebelastung am Patienten auftritt, wenn aufgrund des fehlenden Videosignals die Regelung die Blende der Lichtquelle vollständig öffnen würde. Die Überwachungsschaltung 32 enthält dazu ein vom Horizontal-Synchronsignal getriggertes, nachtriggerbares Monoflop 33 und ein diesem nachgeschaltetes, z.B. negativ flankengetriggertes Monoflop 34. Bei vorhandenem Videosignal liefert diese Überwachungsschaltung keine Ausgangssignale. Erst bei fehlendem Horizontal-Synchronsignal wird mit der negativen Flanke des Ausgangssignals des Monoflops 33 das Monoflop 34 getriggert, und die Überwachungsschaltung 32 liefert als Ausgangssignale einen kurzen und einen längeren Impuls. Der kurze Impuls wird dem Umschalter 30 zugeführt, wodurch dieser auf manuellen Betrieb umschaltet. Der längere Impuls verstellt gleichzeitig im Soll-Ist-Wert-Vergleicher 28 den Soll-Wert so, daß durch das Ausgangssignal des Soll-Ist-Wert-Vergleichers der Motortreiber 12 dafür sorgt, daß die Blende 10 geschlossen wird.

Durch eine weitere Vorrichtung kann die Blendenstellung optisch als Indikation über die abgegebene Lichtmenge angezeigt werden.

## Patentansprüche

1. Einrichtung zur automatischen Regelung der für das Beleuchtungslicht eines Endoskopes vorgesehenen Lichtquelle mittels eines Videosignales, mit einem das Beleuchtungslicht übertragenden, an das zu betrachtende Objekt heranzuführenden Lichtleiter, einem mit einer Videokamera verbundenen Bildleiter, einem Monitor zur Darstellung des von der Videokamera aufgenommenen Bildes sowie einer Signalverarbeitungseinrichtung für das von der Videokamera erzeugte Signal, dadurch gekennzeichnet, daß für die Erzeugung eines Ist-Wertes aus dem Videosignal die Signalverarbeitungseinrichtung (3) einen Verstärker (18) mit variabler Verstärkung, eine Integrationsschaltung (26) und ein Abtast-Halteglied (27) sowie zur Erzeugung des zur Steuerung des Verstärkers (18) benötigten Signals eine Einrichtung (15) zur Erfassung der Zeilenanzahl des auf dem Monitor (7) dargestellten, durch die Videokamera (5) aufgenommenen Videobildes und einen Funktionsbaustein (24) zur Durchführung einer mathematischen Operation umfaßt, wodurch der Ist-Wert unabhängig vom Bildkreisdurchmesser des Endoskopes ist.

2. Einrichtung nach Anspruch 1, dadurch gekennzeichnet, daß ein Wahlschalter (30) für automatische oder manuelle Lichtregelung vorgesehen ist.

3. Einrichtung nach Anspruch 1 oder 2, dadurch gekennzeichnet, daß eine Überwachungsschaltung (32) vorgesehen ist, welche bei fehlendem Videosignal die Lichtquellenregelung auf manuellen Betrieb umschaltet und die Blende der Lichtquelle schließt.

4. Einrichtung nach Anspruch 1, dadurch gekennzeichnet, daß nur die Zeilen gezählt werden, die einen Helligkeitswert aufweisen, der oberhalb eines einstellbaren Grenzwertes liegt.

5. Einrichtung nach einem der Ansprüche 1 bis 3, gekennzeichnet durch Mittel, mit deren Hilfe die von der Lichtquelle (2) abgegebene Lichtmenge aus der Stellung der Blende (10) zur Anzeige bringbar ist.

## Claims

1. A device for the automatic regulation of the light source provided for the illuminating light of an endoscope by means of a video signal, with a photoconductor transferring the illuminating light and bringing it up to the object which is to be observed, with an image conductor connected with a video camera, with a monitor to display the image received by the video camera and with a signal processing device for the signal produced from the video camera, characterised in that for the production of an actual value from the video signal, the signal processing device (3) comprises an amplifier (18) with variable amplification, an integration circuit (26) and a scanning holding element (27) and also for the production of the signal required to control the amplifier (18) a device (15) to pick up the number of lines of the video image represented on the monitor (7) and received by the video camera (5), and a function component (24) to carry out a mathematical operation, whereby the actual value is independent of the image circle diameter of the endoscope.

2. A device according to Claim 1, characterised in that a selector switch (30) is provided for automatic or manual light regulation.

3. A device according to Claim 1 or 2, characterised in that a monitoring circuit (32) is provided, which in the absence of a video signal switches the light source regulating arrangement over to manual operation and closes the shutter of the light source.

4. A device according to Claim 1, characterised in that only the lines are counted which have a brightness value which lies above an adjustable threshold value.

5. A device according to one of Claims 1 to 3, characterised by means with the aid of which the amount of light emitted by the light source (2) is able to be brought into display from the position of the shutter (10).

## Revendications

1. Mécanisme pour le réglage automatique de la source lumineuse prévue pour la lumière d'éclairage d'un endoscope au moyen d'un signal vidéo, comprenant un conducteur optique transmettant la lumière d'éclairage, qui doit être guidé le long de l'objet à observer, un conducteur rigide d'images relié à une caméra vidéo, un moniteur pour représenter l'image captée par la caméra vidéo, ainsi qu'un mécanisme de traitement de signaux pour le signal émis par la caméra vidéo, caractérisé en ce que, pour générer une valeur effective à partir du signal vidéo, le mécanisme de traitement de signaux (3) comprend un amplificateur (18) à gain variable, un circuit d'intégration (26) et un élément d'interrogation-maintien (27), ainsi que, pour l'obtention du signal requis pour la commande de l'amplificateur (18), un mécanisme (15) destiné à l'enregistrement du nombre de lignes de l'image vidéo prise par la caméra vidéo (5) et représentée sur le moniteur (7), ainsi qu'un composant fonctionnel (24) pour effectuer une opération mathématique, par lequel la valeur effective est indépendante du diamètre d'image de l'endoscope.

2. Mécanisme selon la revendication 1, caractérisé en ce qu'on prévoit un commutateur sélectif (30) pour le réglage automatique ou manuel de la lumière.

3. Mécanisme selon la revendication 1 ou 2, caractérisé en ce qu'on prévoit un circuit de surveillance (32) qui, en l'absence d'un signal vidéo, commute le réglage de la source lumineuse à un fonctionnement manuel et ferme l'obturateur de la source lumineuse.

4. Mécanisme selon la revendication 1, caractérisé en ce que, seules sont comptées les lignes qui présentent une valeur de luminosité qui se situe au-dessus d'une valeur limite réglable.

5. Mécanisme selon l'une quelconque des revendications 1 à 3, caractérisé par des moyens à l'aide desquels l'intensité lumineuse émise par la source lumineuse (2) peut être affichée à partir de la position de l'obturateur (10).
